# FASCICULE DE BREVET EUROPEEN

(11) **EP 0 966 949 B1**
(45) Date de publication et mention de la délivrance du brevet: **28.11.2001**
(21) Numéro de dépôt: 99401433.0
(22) Date de dépôt: 11.06.1999
(51) Int. Cl.: A61K 7/06, A61K 7/48

(54) **Composition cosmétique contenant un polysaccharide et un terpolymère acrylique et utilisation de cette composition pour le traitement des matières kératiniques**
Kosmetische Zusammensetzung enthaltend ein Polysaccharid und ein acrylisches Terpolymer und Verwendung dieser Zusammensetzung zur Behandlung von Keratinfasern
Cosmetic composition containing a polysaccharide and an acrylic terpolymer and use of said composition for treating keratinic materials

(30) Priorité: 15.06.1998 FR 9807515
(43) Date de publication de la demande: 29.12.1999
(73) Titulaire: L'OREAL, 75008 Paris (FR)
(72) Inventeur: Dupuis, Christine, 75018 Paris (FR)
(74) Mandataire: Casalonga, Axel

(56) Documents cités:
- EP-A- 0 824 914

## Description

La présente invention concerne des compositions cosmétiques contenant en association, au moins un polysaccharide et un terpolymère acrylique ainsi que l'utilisation de ces compositions pour le traitement des matières kératiniques, en particulier la peau et les cheveux.

Les polymères de type polysaccharides conduisent à un très bon effet coiffant sur cheveux. Cependant, la plupart ne sont pas épaississants, ou s'ils le sont, donnent des gels de viscosité insuffisante ou de texture filante.

Il est alors nécessaire, lorsqu'on souhaite les présenter sous forme de gel de coiffage ou de soin, de les associer avec un épaississant qui leur donne une texture adéquate tout en conservant les bonnes performances cosmétiques de ces produits.

Les épaississants les plus couramment utilisés sont généralement à base de polymères acryliques réticulés comme les produits vendus sous la dénomination "CARBOPOL" par la société GOODRICH et ceux vendus sous la dénomination "SYNTHALEN K" par la société 3V. Ces polymères, utilisés en combinaison avec des polysaccharides, conduisent le plus souvent à des gels de texture non satisfaisante et qui ne sont pas non plus satisfaisants sur le plan des résultats cosmétiques car ils ne confèrent pas aux cheveux de bonnes propriétés de démêlage et de douceur ; ils n'ont pas non plus un bon pouvoir fixant. On connaît d'autres polymères épaississants et/ou gélifiants comportant dans leur chaîne une partie hydrophile et une partie hydrophobe constituée d'une chaîne grasse comme le produit "PEMULEN TR1" commercialisé par la société GOODRICH ou les polymères "ACRYSOL" commercialisés par la société ROHM & HAAS. Le polymère "PEMULEN TR1", utilisé en association avec des polysaccharides, ne conduit pas à un gel de texture satisfaisante et ne donne pas sur le plan cosmétique des résultats satisfaisants, notamment en ce qui concerne le pouvoir fixant. Le polymère "ACRYSOL 44", en association avec un polysaccharide, conduit à un produit très fluide et trouble.

La demanderesse a découvert de manière surprenante qu'en utilisant une nouvelle famille de polymères épaississants et/ou gélifiants et en les associant à des polysaccharides, on pouvait obtenir des formulations cosmétiques présentant une viscosité satisfaisante à un pH relativement peu élevé, qui sont non pâteuses, qui s'étalent bien sur la peau et les cheveux et qui leur confèrent de bonnes propriétés de douceur et de démêlage tout en ayant de bonnes propriétés fixantes.

La présente invention a donc pour objet des compositions cosmétiques contenant, dans un support aqueux cosmétiquement acceptable, au moins un polysaccharide et un terpolymère acrylique que l'on définira plus en détail dans la suite de la description.

Ce polymère permet notamment de préparer des compositions aqueuses ou hydro-organiques contenant des solvants cosmétiquement acceptables, rincées ou non rincées, allant des produits légèrement gélifiés aux sticks ou bâtonnets solides.

Les avantages de ce terpolymère sont d'être stable en milieu électrolyte et d'avoir un très bon pouvoir épaississant à pH égal ou supérieur à 5,5 permettant d'atteindre un bon niveau de viscosité et de pouvoir utiliser des concentrations élevées en alcool.

Ce polymère, utilisé en association avec au moins un polysaccharide, permet de réaliser des produits gélifiés non pâteux, ayant une bonne facilité d'étalement, doux à l'application et ayant un bon pouvoir fixant.

Il permet également d'améliorer l'effet de conditionnement des polysaccharides sur les cheveux, notamment leur toucher.

Le terpolymère acrylique utilisé conformément à l'invention est soluble ou gonflable dans les alcalis. Il est caractérisé par le fait qu'il comprend :
a) 20 à 70% en poids d'un acide carboxylique à insaturation α, β-monoéthylénique ;
b) au moins 20% en poids d'un monomère à insaturation monoéthylénique non tensio-actif différent de a) et
c) 0,5 à 60% en poids, d'un monomère uréthane non-ionique qui peut être obtenu par réaction d'un tensio-actif non-ionique monohydrique avec un monoisocyanate à insaturation monoéthylénique.

De préférence, le terpolymère comprend les monomères a), b) et c) à raison de 25 à 55 %, 30 à 65 et respectivement au moins 10% en poids.

L'acide carboxylique à insaturation α, β-monoéthylénique a) peut être choisi parmi de nombreux acides et en particulier l'acide acrylique, l'acide méthacrylique, l'acide itaconique et l'acide maléique. L'acide méthacrylique est préféré. Une large proportion d'acide est essentielle pour donner une structure polymère qui se solubilise et donne un épaississant par réaction avec un composé alcalin comme l'hydroxyde de sodium, les alcanolamines, l'amino méthyl propanol, l'amino méthyl propanediol.

Le terpolymère doit aussi contenir une proportion importante indiquée ci-dessus d'un monomère b) à insaturation monoéthylénique qui n'a pas de propriété tensio-active. Les monomères préférés sont ceux qui donnent des polymères insolubles dans l'eau lorsqu'ils sont homopolymérisés et sont illustrés par les acrylates et méthacrylates d'alkyle en C₁-C₄ comme l'acrylate de méthyle, l'acrylate d'éthyle, l'acrylate de butyle ou les méthacrylates correspondants. Les monomères plus particulièrement préférés sont les acrylates de méthyle et d'éthyle. D'autres monomères pouvant être utilisés sont le styrène, le vinyltoluène, l'acétate de vinyle, l'acrylonitrile et le chlorure de vinylidène. Les monomères non réactifs sont préférés, ces monomères étant ceux dans lesquels le groupe éthylénique unique est le seul groupe réactif dans les conditions de la polymérisation. Cependant, des monomères qui contiennent des groupes réactifs sous l'action de la chaleur peuvent être utilisés dans certaines situations, comme l'acrylate d'hydroxyéthyle.

Les tensio-actifs non-ioniques monohydriques utilisés pour obtenir le monomère uréthane non-ionique c) sont bien connus et sont généralement des composés hydrophobes alcoxylés contenant un oxyde d'alkylène formant la partie hydrophile de la molécule. Les hydrophobes sont généralement constitués par un alcool aliphatique ou un alkylphénol dans lesquels une chaîne carbonée contenant au moins six atomes de carbone constitue la partie hydrophobe du tensio-actif.

Les tensio-actifs non ioniques monohydriques préférés ont pour formule : 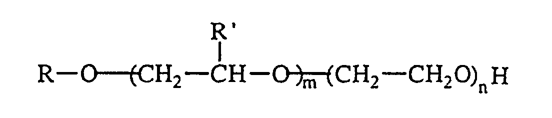 dans laquelle R est un groupe alkyle en C₆-C₃₀ ou aralkyle en C₈-C₃₀, R' est un groupe alkyle en C₁-C₄, n est un nombre moyen allant de 5 à 150 et m est un nombre moyen allant de 0 à 50, à la condition que n soit au moins aussi grand que m et que n + m = 5-150.

A titre de groupes alkyle en C₆-C₃₀ préférés, on peut citer les radicaux dodécyle et alkyle en C₁₈-C₂₆. A titre de groupes aralkyle, on peut citer plus particulièrement les groupes alkyl (C₈-C₁₃) phényle. Le groupe R' préféré est le groupe méthyle.

Le monoisocyanate à insaturation monoéthylénique utilisé pour former le monomère uréthane non-ionique c) peut être choisi parmi des composés très variés. On peut utiliser un composé contenant toute insaturation copolymérisable telle qu'une insaturation acrylique ou méthacrylique. On peut aussi utiliser une insaturation allylique conférée par l'alcool allylique. Le monoisocyanate monoéthylénique préféré est l'α,α-diméthyl-m-isopropényl-benzylisocyanate.

Le terpolymère acrylique défini ci-dessus est obtenu par copolymérisation en émulsion aqueuse des composants a), b) et c) qui est tout à fait usuelle et décrite dans la demande de brevet EP-A-0 173 109.

A titre de terpolymères pouvant être utilisés selon l'invention, on peut citer les produits de réaction de l'acide méthacrylique comme composant a), de l'acrylate d'éthyle comme composant b) et d'un macromonomère uréthane non-ionique comme composant c), ayant la structure suivante : 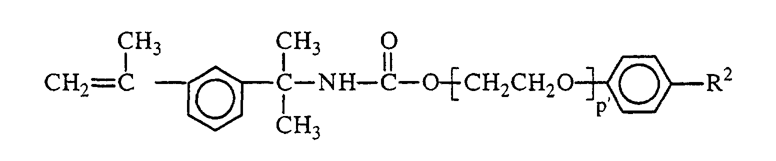 dans laquelle p' va de 6 à 150 et est de préférence égal à 30 et R² est un radical alkyle en C₈-C₁₃, tels que celui décrit dans l'exemple 3 de la demande de brevet EP-A-0 173 109.

Le terpolymère acrylique préféré utilisé selon l'invention est obtenu à partir d'acide méthacrylique comme composant a), d'acrylate de méthyle comme composant b) et d'un macromonomère uréthane non-ionique comme composant (c), ayant la structure suivante : 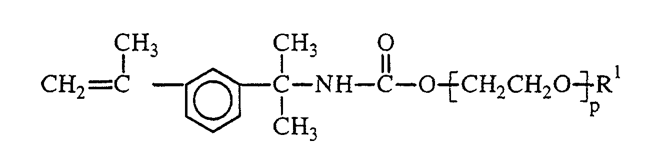 dans laquelle p va de 6 à 150 et R¹ est un radical alkyle en C₁₈-C₂₆, de préférence en C₂₀-C₂₄ linéaire, d'origine végétale tel que le radical docosyle.

Le terpolymère acrylique est présent dans les compositions cosmétiques de l'invention dans des concentrations allant de 0,01 à 20% en poids par rapport au poids total de la composition et préférentiellement de 0,1 à 10% en poids.

Selon la présente invention, il est possible d'utiliser tout polysaccharide ou tout dérivé de polysaccharide connu en soi, qu'il s'agisse d'homopolysaccharide ou d'hétéropolysaccharide, d'origine animale, végétale, microbienne. bactérienne ou synthétique.

D'une manière générale, les polysaccharides et leurs dérivés utilisables dans le cadre de la présente invention sont ceux qui sont notamment décrits dans "Encyclopedia of Chemical Technology, Kirk-Othmer, Third Edition, 1982, volume 3, pp. 896-900, et volume 15, pp 439-458", dans "Polymers in Nature, par E. A. MacGREGOR et C. T. GREENWOOD, Editions John Wiley & Sons, Chapter 6, pp 240-328, 1980" et dans l'Industrial Gums - Polysaccharides and their Derivatives, Edité par Roy L. WHISTLER, Second Edition, Edition Academic Press Inc.",

Selon la présente invention, on peut bien entendu soit utiliser un seul et même polysaccharide et/ou l'un de ses dérivés, ou, au contraire, mettre en oeuvre plusieurs polysaccharides et/ou dérivés de polysaccharides différents.

A titre d'exemples de polysaccharides ou de dérivés de polysaccharides convenant à la mise en oeuvre de l'invention, on peut notamment citer les glucanes, les amidons modifiés ou non (tels que ceux tirés par exemple de céréales comme le blé, le maïs ou le riz, de légumes comme le pois blond, de tubercules comme les pommes de terre ou le manioc), l'amylose, l'amylopectine, le glycogène les dextranes, les β-glucanes, les celluloses et leurs dérivés (méthylcelluloses, hydroxyalkylcelluloses, éthylhydroxyéthylcelluloses, carboxyméthylcelluloses), les fructosanes, l'inuline, la levane, les mannanes, les xylanes, les arabanes, les galactanes, les galacturonanes, la chitine, les glucoronoxylanes, les arabinoxylanes, les xyloglucanes, les galactomannanes, les glucomannanes, les acides pectiques et les pectines, l'acide alginique et les alginates, les arabinogalactanes, les carraghénines, les agars, les glycosaminoglucanes, les gommes arabiques, les gommes Tragacanthe, les gommes Ghatti, les gommes Karaya, les gommes de caroube, les gommes de guar et les gommes de xanthane.

Les polysaccharides préférés pour être utilisés selon l'invention sont des gommes de guar.

Selon l'invention, on peut utiliser les gommes de guar non-ioniques chimiquement modifiées ou non modifiées.

Les gommes de guar non-ioniques non modifiées sont par exemple les produits vendus sous la dénomination VIDOGUM GH 175 par la société UNIPECTINE et sous la dénomination JAGUAR C par la société MEYHALL.

Les gommes de guar non-ioniques modifiées utilisables selon l'invention sont de préférence modifiées par des groupements hydroxyalkyle en C₁-C₆.

Parmi les groupements hydroxyalkyle, on peut mentionner à titre d'exemple, les groupements hydroxyméthyle, hydroxyéthyle, hydroxypropyle et hydroxybutyle.

Ces gommes de guar sont bien connues de l'état de la technique et peuvent par exemple être préparées en faisant réagir des oxydes d'alcènes correspondants, tels que par exemple des oxydes de propylène, avec la gomme de guar de façon à obtenir une gomme de guar modifiée par des groupements hydroxypropyle.

Le taux d'hydroxyalkylation, qui correspond au nombre de molécules d'oxyde d'alkylène consommées par le nombre de fonctions hydroxyle libres présentes sur la gomme de guar, varie de préférence de 0,4 à 1,2.

De telles gommes de guar non-ioniques éventuellement modifiées par des groupements hydroxyalkyle sont par exemple vendues sous les dénominations commerciales JAGUAR HP8, JAGUAR HP60 et JAGUAR HP120, JAGUAR DC293 et JAGUAR HP105 par la société MEYHALL, ou sous la dénomination GALACTASOL 4H4FD2 par la société AQUALON.

Les polysaccharides sont utilisés dans les compositions de l'invention dans des proportions comprises entre 0,01 et 20% en poids et de préférence entre 0,1 et 10% en poids par rapport au poids total de la composition.

Les compositions selon l'invention contiennent un milieu aqueux cosmétiquement acceptable. Elles présentent un pH pouvant aller de 3,5 à 11, de préférence compris entre 5,5 et 11 et encore plus préférentiellement entre 5,5 et 8,5.

Le milieu cosmétiquement acceptable des compositions selon l'invention est plus particulièrement constitué d'eau et éventuellement de solvants organiques cosmétiquement acceptables.

Les solvants organiques peuvent représenter de 0,5 à 90% du poids total de la composition. Ils peuvent être choisis dans le groupe constitué par les solvants organiques hydrophiles, les solvants organiques lipophiles, les solvants amphiphiles ou leurs mélanges.

Parmi les solvants organiques hydrophiles, on peut citer par exemple les mono-alcools inférieurs linéaires ou ramifiés ayant de 1 à 8 atomes de carbone, les polyéthylène glycols ayant de 6 à 80 unités d'oxyde d'éthylène et les polyols.

Comme solvants organiques amphiphiles, on peut citer les dérivés de polypropylène glycol (PPG) comme les esters de polypropylène glycol et d'acide gras, de PPG et d'alcool gras comme le PPG-23 oléyl éther et le PPG-36 oléate.

Comme solvants organiques lipophiles, on peut citer par exemple les esters gras tels que l'adipate de diisopropyle, l'adipate de dioctyle, les benzoates d'alkyle, le malate de dioctyle.

Afin que les compositions cosmétiques de l'invention soient plus agréables à utiliser (plus douces à l'application, plus nourrissantes, plus émollientes), il est possible d'ajouter une phase grasse dans le milieu de ces compositions.

La phase grasse peut représenter jusqu'à 50% du poids total de la composition.

Cette phase grasse peut comporter une huile ou une cire ou leurs mélanges, et peut comprendre également des acides gras, des alcools gras et des esters d'acides gras. Les huiles peuvent être choisies parmi les huiles animales, végétales, minérales ou de synthèse et notamment parmi l'huile de vaseline, l'huile de paraffine, les isoparaffines, les poly-α-oléfines, les huiles fluorées et perfluorées. De même, les cires peuvent être choisies parmi les cires animales, fossiles, végétales, minérales ou de synthèse connues en soi.

Les compositions de l'invention peuvent contenir des adjuvants habituels dans le domaine cosmétique comme d'autres gélifiants et/ou épaississants classiques; des émulsionnants; des tensio-actifs; des agents hydratants ; des émollients; des filtres solaires ; des actifs hydrophiles ou lipophiles comme des céramides ; des agents anti-radicaux libres ; des séquestrants ; des antioxydants ; des conservateurs; des agents alcalinisants ou acidifiants; des parfums ; des charges ; des matières colorantes, des silicones volatiles ou non, modifiées ou non ; des réducteurs. Les quantités de ces différents adjuvants sont celles classiquement utilisées dans les domaines considérés.

Bien entendu, le spécialiste veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Les compositions selon l'invention peuvent se présenter sous toutes les formes appropriées pour une application topique, notamment sous forme de solutions du type lotion, sous forme de gels aqueux ou hydroalcooliques, sous forme de dispersions vésiculaires, sous forme d'émulsions simples ou complexes ((H/E, E/H, H/E/H ou E/H/E) et être de consistance liquide, semi-liquide ou solide telles que des laits, des crèmes des gels, des gels-crèmes, des pâtes, des sticks et éventuellement être conditionnées en aérosol et se présenter sous la forme de mousses ou de sprays. Ces compositions sont préparées selon les méthodes usuelles.

Les compositions selon l'invention peuvent être utilisées comme produits capillaires rincés ou non-rincés, notamment pour le lavage, la coloration, le soin, le conditionnement, le défrisage, le maintien de la coiffure ou la mise en forme permanente ou non des cheveux.

Elles peuvent être des produits de coiffage tels que des lotions de mise en plis, des lotions pour le brushing, des compositions de fixation et de coiffage. Les lotions peuvent être conditionnées sous diverses formes notamment dans des vaporisateurs, des flacons pompes ou dans des récipients aérosols afin d'assurer une application de la composition sous forme vaporisée ou sous forme de mousse. De telles formes de conditionnement sont indiquées, par exemple, lorsqu'on souhaite obtenir un spray ou une mousse pour la fixation ou le traitement des cheveux.

Les compositions de l'invention peuvent être également des shampooings, des compositions à rincer ou non, à appliquer avant ou après un shampooing, une coloration, une décoloration, une permanente ou un défrisage.

Les compositions de l'invention peuvent être également utilisées comme produits de soin ou d'hygiène tels que des crèmes de protection, de traitement ou de soin pour le visage, pour les mains ou pour le corps, des laits corporels de protection ou de soin, des lotions, gels ou mousses pour le soin ou le nettoyage de la peau.

Les compositions de l'invention peuvent être également utilisées comme compositions antisolaires.

Les compositions peuvent également consister en des préparations solides constituant des savons ou des pains de nettoyage.

Les compositions de l'invention peuvent être également utilisées comme produits de soin bucco-dentaire tels que des pâtes dentifrices, des bains de bouche.

Les compositions peuvent être des produits pour le maquillage tels que des crèmes pour le visage, des fonds de teint, des mascaras, des eye-liners, des rouges à lèvres, des vernis à ongles.

Un autre objet de l'invention est un procédé de traitement non-thérapeutique cosmétique de la peau, du cuir cheveu, des cheveux, des cils, des sourcils, des ongles ou des muqueuses, caractérisé par le fait qu'on applique sur le support kératinique une composition telle que définie ci-dessus, selon la technique d'utilisation habituelle de cette composition, par exemple application de crèmes, de gels, de sérums, de lotions, de laits sur la peau, le cuir chevelu ou les muqueuses.

Les exemples suivants illustrent l'invention sans présenter un caractère limitatif.

### EXEMPLE 1 : Gel de coiffage non-rincé

- Terpolymère acide méthacrylique/méthyl acrylate/ diméthyl-méta-isopropényl-benzyl-uréthane d'alcool béhénylique éthoxylé (40 OE) en dispersion aqueuse à 25% 0,5 g MA
- Gomme d'hydroxypropylguar vendue sous la dénomination JAGUAR HP105 par la société MEYHALL 0,5 g MA
- 2-amino 2-méthyl propanol-1 (AMP) pH ajusté à 7.5 qs
- Parfum, conservateur, colorant q.s
- Eau déminéralisée qsp 100 g
MA signifie matière active.

On obtient un gel épais, non pâteux et s'étalant très bien sur les cheveux. Ce gel donne aux cheveux un toucher doux et une bonne aptitude au démêlage et possède un bon pouvoir fixant.

Si on remplace le terpolymère ci-dessus par la même quantité de polyuréthane "ACRYSOL 44" de ROHM et HAAS, on obtient un produit très fluide et trouble.

Si on remplace le terpolymère par le copolymère acide acrylique/acrylate d'alkyle en C₁₀/C₃₀ réticulé "PEMULEN TR1" vendu par GOODRICH, on obtient un gel légèrement pâteux, s'étalant moins bien et ayant un pouvoir fixant médiocre.

### EXEMPLE 2 : Gel solaire haute protection

- 4 tert-butyl 4'-méthoxy-dibenzoylméthane ("PARSOL 1789" vendu par la société ROCHE) 2 g
- Acide benzène-1,4-di(3-méthylidène-10-camphosulfonique) en solution aqueuse à 33% 3 g
- 2-cyano-3,3-diphénylacrylate de 2-éthylhexyle ("UVINUL N 539" vendu par la société BASF) 10 g
- Terpolymère acide méthacrylique/méthyl acrylate/ diméthyl méta-isopropényl-benzyl-uréthane d'alcool béhénylique éthoxylé (40 OE) en dispersion aqueuse à 25% 0,55g MA
- Gomme de xanthane vendue sous la dénomination "RHODICARE S" par la société RHONE POULENC 0,8 g
- 2,2,4,4,6,6,8-heptaméthylnonane 4 g
- Glycérol 6 g
- Propylèneglycol 6 g
- Acide éthylène-diamine-tétra (méthylène phosphonique), sel pentasodique en solution aqueuse à 33% 0,3 g
- Triéthanolamine 0,92g
- Alcool éthylique à 96° dénaturé 4,5 g
- Eau déminéralisée stérilisée qsp 100 g

On obtient un gel onctueux s'étalant bien sur la peau.

## Revendications

1. Composition cosmétique destinée au traitement des matières kératiniques, **caractérisée par le fait qu'**elle comprend, dans un support aqueux cosmétiquement acceptable, au moins un polysaccharide et un terpolymère acrylique comprenant :
a) 20 à 70% en poids, d'un acide carboxylique à insaturation α, β-monoéthylénique ;
b) au moins 20% en poids, d'un monomère à insaturation monoéthylénique non tensio-actif différent de a) et
c) 0,5 à 60% en poids, d'un monomère uréthane non-ionique qui peut être obtenu par réaction d'un tensio-actif non-ionique monohydrique avec un monoisocyanate à insaturation monoéthylénique.

2. Composition selon la revendication 1, **caractérisée par le fait que** l'acide carboxylique à insaturation α, β-monoéthylénique a) est choisi parmi l'acide acrylique, l'acide méthacrylique, l'acide itaconique et l'acide maléique.

3. Composition selon la revendication 2, **caractérisée par le fait que** l'acide carboxylique à insaturation α, β-monoéthylénique a) est l'acide méthacrylique.

4. Composition selon l'une quelconque des revendications 1 à 3, **caractérisée par le fait que** le monomère à insaturation monoéthylénique non tensio-actif b) est choisi parmi les acrylates et méthacrylates d'alkyle en C₁-C₄, le styrène, le vinyltoluène, l'acétate de vinyle, l'acrylonitrile et le chlorure de vinylidène.

5. Composition selon la revendication 4, **caractérisée par le fait que** le monomère à insaturation monoéthylénique non tensio-actif est l'acrylate de méthyle ou d'éthyle.

6. Composition selon l'une quelconque des revendications 1 à 5, **caractérisée par le fait que** le tensio-actif non-ionique monohydrique utilisable pour obtenir le monomère uréthane non-ionique c) a pour formule : 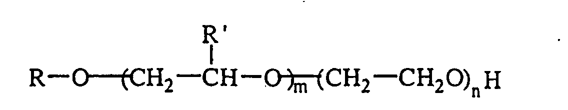 dans laquelle R est un groupe alkyle en C₆-C₃₀ ou aralkyle en C₈-C₃₀, R' est un groupe alkyle en C₁-C₄, n est un nombre moyen allant de 5 à 150 et m est un nombre moyen allant de 0 à 50, à la condition que n soit au moins aussi grand que m et que n + m = 5-150.

7. Composition selon la revendication 6, **caractérisée par le fait que** R est choisi parmi les radicaux dodécyle, alkyle en C₁₈-C₂₆ et alkyl (C₈-C₁₃) phényle et m= 0.

8. Composition selon l'une quelconque des revendications 1 à 7, **caractérisée par le fait que** le monoisocyanate à insaturation monoéthylénique utilisable pour former le monomère uréthane non-ionique c) est l'α,α-diméthyl-m-isopropényl-benzyl-isocyanate.

9. Composition selon l'une quelconque des revendications 1 à 8, **caractérisée par le fait que** le terpolymère acrylique est obtenu en dispersion aqueuse à partir d'acide méthacrylique comme composant a), d'acrylate de méthyle comme composant b) et d'un macromonomère uréthane non-ionique de structure suivante comme composant c): 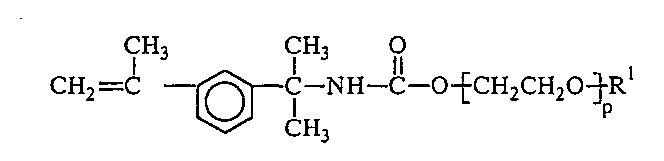 dans laquelle p va de 6 à 150 et R¹ est un radical alkyle en C₁₈-C₂₆, de préférence en C₂₀-C₂₄ linéaire, d'origine végétale, tel que le radical docosyle.

10. Composition selon l'une quelconque des revendications 1 à 9, **caractérisée par le fait que** le terpolymère acrylique est présent dans des concentrations allant de 0,01 à 20% en poids, et de préférence de 0,1 à 10 % en poids, par rapport au poids total de la composition.

11. Composition selon l'une quelconque des revendications 1 à 10, **caractérisée par le fait que** les polysaccharides sont choisis parmi les glucanes, les amidons modifiés ou non (tels que ceux tirés par exemple de céréales comme le blé, le maïs ou le riz, de légumes comme le pois blond, de tubercules comme les pommes de terre ou le manioc), l'amylose, l'amylopectine, le glycogène, les dextranes, les β-glucanes, les celluloses et leurs dérivés (méthylcelluloses, hydroxyalkylcelluloses, éthylhydroxyéthyl-celluloses, carboxyméthylcelluloses), les fructosanes, l'inuline, la levane, les mannanes, les xylanes, les arabanes, les galactanes, les galacturonanes, la chitine, les glucoronoxylanes, les arabinoxylanes, les xyloglucanes, les galactomannanes, les glucomannanes, les acides pectiques et les pectines, l'acide alginique et les alginates, les arabinogalactanes, les carraghénines, les agars, les glycosaminoglucanes, les gommes arabiques, les gommes Tragacanthe, les gommes Ghatti, les gommes Karaya, les gommes de caroube, les gommes de guar et les gommes de xanthane.

12. Composition selon la revendication 11, **caractérisée par le fait que** les polysaccharides sont des gommes de guar non-ioniques chimiquement modifiées ou non-modifiées.

13. Composition selon la revendication 12, **caractérisée par le fait que** les gommes de guar sont modifiées par des groupements hydroxyalkyle en C₁-C₆.

14. Composition selon la revendication 13, **caractérisée par le fait qu'**elle contient une gomme de guar modifiée par un groupement hydroxypropyle.

15. Composition selon l'une quelconque des revendications 1 à 14, **caractérisée par le fait que** le ou les polysaccharides sont présents dans des concentrations allant de 0,01 à 20% en poids, et de préférence de 0,1 à 10% en poids, par rapport au poids total de la composition.

16. Composition selon l'une quelconque des revendications 1 à 15, **caractérisé par le fait qu'**elle présente un pH allant de 3,5 à 11, de préférence de 5,5 à 11 et encore plus préférentiellement de 5,5 à 8,5.

17. Composition selon l'une quelconque des revendications 1 à 16, **caractérisée par le fait que** le milieu aqueux cosmétiquement acceptable est constitué d'eau ou d'eau et au moins un solvant organique choisi dans le groupe constitué par les solvants organiques hydrophiles, lipophiles, amphiphiles ou leurs mélanges.

18. Composition selon l'une quelconque des revendications 1 à 17, **caractérisée par le fait qu'**elle comprend en outre au moins un adjuvant cosmétique usuel choisi parmi les corps gras, les gélifiants et/ou épaississants classiques, les tensio-actifs, les agents hydratants, les émollients, les filtres solaires, les actifs hydrophiles ou lipophiles comme les céramides, les agents anti-radicaux libres, les séquestrants, les antioxydants, les conservateurs, les agents alcalinisants ou acidifiants, les parfums, les charges, les matières colorantes, les silicones et les réducteurs.

19. Composition selon l'une quelconque des revendications 1 à 18, **caractérisée par le fait qu'**elle se présente sous forme d'émulsion, de lotion, de gel, de dispersion vésiculaire, de pâte, de bâtonnet solide ou est conditionnée en aérosol et se présente sous forme de mousse ou de spray.

20. Composition selon l'une quelconque des revendications 1 à 19, **caractérisée par le fait qu'**elle est utilisée comme produit capillaire rincé ou non rincé pour le lavage, la teinture, le soin, le conditionnement, le défrisage, le maintien de la coiffure ou la mise en forme permanente ou non des cheveux, ou comme composition antisolaire.

21. Composition selon l'une quelconque des revendications 1 à 20, **caractérisé par le fait que** le terpolymère comprend les monomères a), b) et c) à raison de 25 à 55%, 30 à 65% et respectivement au moins 10% en poids.

22. Procédé de traitement non-thérapeutique cosmétique pour la protection de la peau, du cuir chevelu, des cheveux, des cils, des sourcils, des ongles et des muqueuses, **caractérisé par le fait qu'**on applique sur ces derniers une quantité efficace d'une composition telle que définie selon l'une quelconque des revendications 1 à 21.

## Patentansprüche

1. Kosmetische Zusammensetzung, die zur Behandlung von Keratinsubstanzen vorgesehen ist, **dadurch gekennzeichnet, daß** sie in einem kosmetisch akzeptablen wäßrigen Träger mindestens ein Polysaccharid und ein Acrylterpolymer enthält, welches aufweist:
a) 20 bis 70 Gew.-% einer Carbonsäure mit α,β-monoethylenischer Doppelbindung;
b) mindestens 20 Gew.-% eines Monomers mit monethylenischer Doppelbindung, das kein grenzflächenaktiver Stoff und von a) verschieden ist, und
c) 0,5 bis 60 Gew.-% eines nichtionischen Urethanmonomers, das durch Umsetzung eines nichtionischen grenzflächenaktiven Stoffes mit einer Hydroxygruppe mit einem Monoisocyanat mit monoethylenischer Doppelbindung erhalten werden kann.

2. Zusammensetzung nach Anspruch 1, **dadurch gekennzeichnet, daß** die Carbonsäure mit α,β-monoethylenischer Doppelbindung
a) unter Acrylsäure, Methacrylsäure, Itaconsäure und Maleinsäure ausgewählt ist.

3. Zusammensetzung nach Anspruch 2, **dadurch gekennzeichnet, daß** die Carbonsäure mit α,β-monoethylenischer Doppelbindung
a) die Methacrylsäure ist.

4. Zusammensetzung nach einem der Ansprüche 1 bis 3, **dadurch gekennzeichnet, daß** das Monomer mit monoethylenischer Doppelbindung b), das kein grenzflächenaktiver Stoff ist, unter den C₁₋₄-Alkylacrylaten und C₁₋₄-Alkylmethacrylaten, Styrol, Vinyltoluol, Vinylacetat, Acrylnitril und Vinylidenchlorid ausgewählt ist.

5. Zusammensetzung nach Anspruch 4, **dadurch gekennzeichnet, daß** das Monomer mit monoethylenischer Doppelbindung, das kein grenzflächenaktiver Stoff ist, Methylacrylat oder Ethylacrylat ist.

6. Zusammensetzung nach einem der Ansprüche 1 bis 5, **dadurch gekennzeichnet, daß** der zur Herstellung des nichtionischen Urethanmonomers c) verwendbare nichtionische grenzflächenaktive Stoff mit einer Hydroxygruppe die folgende Formel aufweist: 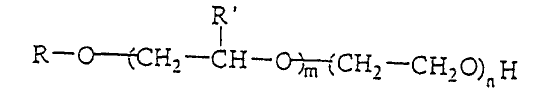 worin R eine C₆₋₃₀-Alkylgruppe oder C₈₋₃₀-Aralkylgruppe, R' eine C₁₋₄-Alkylgruppe, n eine Zahl von 5 bis 150 und m eine Zahl von 0 bis 50 bedeutet, mit der Maßgabe, daß n mindestens so groß wie m ist und n + m = 5 - 150.

7. Zusammensetzung nach Anspruch 6, **dadurch gekennzeichnet, daß** R unter Dodecyl, C₁₈₋₂₆-Alkyl und C₈₋₁₃-Alkylphenyl ausgewählt ist und m = 0.

8. Zusammensetzung nach einem der Ansprüche 1 bis 7, **dadurch gekennzeichnet, daß** das Monoisocyanat mit monoethylenischer Doppelbindung, das zur Bildung des nichtionischen Urethanmonomers c) verwendbar ist, das α,α-Dimethyl-m-isopropenylbenzylisocyanat ist.

9. Zusammensetzung nach einem der Ansprüche 1 bis 8, **dadurch gekennzeichnet, daß** das Acrylterpolymer in wäßriger Dispersion ausgehend von Methacrylsäure als Komponente a), Methylacrylat als Komponente b) und einem nichtionischen Urethanmakromer der folgenden Struktur als Komponente c) hergestellt wird: 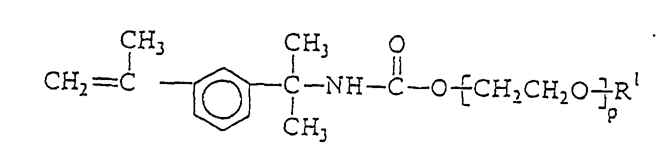 wobei p im Bereich von 6 bis 150 liegt und R¹ eine Alkylgruppe mit 18 bis 26 Kohlenstoffatomen und vorzugsweise eine geradkettige Alkylgruppe mit 20 bis 24 Kohlenstoffatomen pflanzlicher Herkunft ist, wie Docosyl.

10. Zusammensetzung nach einem der Ansprüche 1 bis 9, **dadurch gekennzeichnet, daß** das Acrylterpolymer in Konzentrationen von 0,01 bis 20 Gew.-% und vorzugsweise 0,1 bis 10 Gew.-%, bezogen auf das Gesamtgewicht der Zusammensetzung, vorliegt.

11. Zusammensetzung nach einem der Ansprüche 1 bis 10, **dadurch gekennzeichnet, daß** die Polysaccharide ausgewählt sind unter: Glucanen, Stärkeverbindungen, die gegebenenfalls modifiziert sind (wie Stärkeverbindungen, die beispielsweise aus Cerealien, wie Weizen, Mais oder Reis, Früchten, wie Erbse, und Knollen, wie Kartoffel oder Maniok, gewonnen wurden), Amylose, Amylopektin, Glykogen, Dextranen, β-Glucanen, Cellulosen und ihren Derivaten (Methylcellulosen, Hydroxyalkylcellulosen, Ethylhydroxyethylcellulosen, Carboxymethylcellulosen), Fructosanen, Inulin, Levan, Mannanen, Xylanen, Arabanen, Galactanen, Galacturonanen, Chitin, Glucuronoxylanen, Arabinoxylanen, Xyloglucanen, Galactomannanen, Glucomannanen, Pektinsäuren und Pektinen, Alginsäure und Alginaten, Arabinogalactanen, Carrageenanen, Agar, Glykosaminoglykanen, Gummi arabicum, Tragant, Ghatti Gummi, Karayagummi, Johannisbrotkernmehl, Guargummi und Xanthangummi.

12. Zusammensetzung nach Anspruch 11, **dadurch gekennzeichnet, daß** die Polysaccharide chemisch modifizierte oder nicht modifizierte nichtionische Guargummen sind.

13. Zusammensetzung nach Anspruch 12, **dadurch gekennzeichnet, daß** die Guargummen mit C₁₋₆-Hydroxyalkylgruppen modifiziert sind.

14. Zusammensetzung nach Anspruch 13, **dadurch gekennzeichnet, daß** sie ein mit Hydroxypropyl modifiziertes Guargummi enthält.

15. Zusammensetzung nach einem der Ansprüche 1 bis 14, **dadurch gekennzeichnet, daß** das Polysaccharid oder die Polysaccharide in Konzentrationen von 0,01 bis 20 Gew.-% und vorzugsweise 0,1 bis 10 Gew.-%, bezogen auf das Gesamtgewicht der Zusammensetzung, vorliegen.

16. Zusammensetzung nach einem der Ansprüche 1 bis 15, **dadurch gekennzeichnet, daß** sie einen pH-Wert von 3,5 bis 11, vorzugsweise 5,5 bis 11 und noch bevorzugter 5,5 bis 8,5 aufweist.

17. Zusammensetzung nach einem der Ansprüche 1 bis 16, **dadurch gekennzeichnet, daß** das kosmetisch akzeptable Medium aus Wasser oder Wasser und mindestens einem organischen Lösungsmittel besteht, das unter den hydrophilen, lipophilen oder amphiphilen organischen Lösungsmitteln oder deren Gemischen ausgewählt ist.

18. Zusammensetzung nach einem der Ansprüche 1 bis 17, **dadurch gekennzeichnet, daß** sie mindestens einen herkömmlichen kosmetischen Zusatzstoff enthält, der unter den Fettsubstanzen, herkömmlichen Gelbildnern und/oder Verdickungsmitteln, grenzflächenaktiven Stoffen, Hydratisierungsmitteln, Emollentien, Sonnenschutzfiltern, hydrophilen oder lipophilen Wirkstoffen, wie Ceramiden, Mitteln gegen freie Radikale, Maskierungsmitteln, Antioxidantien, Konservierungsmitteln, Alkalisierungsmitteln oder Ansäuerungsmitteln, Parfums, Füllstoffen, Farbmitteln, Siliconen und Reduktionsmitteln ausgewählt ist.

19. Zusammensetzung nach einem der Ansprüche 1 bis 18, **dadurch gekennzeichnet, daß** sie als Emulsion, Lotion, Gel, Vesikeldispersion, Paste oder fester Stift vorliegt oder als Aerosol konfektioniert ist und in Form von Schaum oder Spray vorliegt.

20. Zusammensetzung nach einem der Ansprüche 1 bis 19, **dadurch gekennzeichnet, daß** sie als Produkt für das Haar, das ausgespült wird oder im Haar verbleibt, zum Waschen, Färben, Pflegen, Konditionieren, Entkräuseln der Haare und für den Halt der Frisur oder die Formgebung der Haare, die dauerhaft oder nicht dauerhaft sein können, verwendet wird.

21. Zusammensetzung nach einem der Ansprüche 1 bis 20, **dadurch gekennzeichnet, daß** das Terpolymer die Monomere a), b) und c) in Mengenanteilen von 25 bis 55 Gew.-%, 30 bis 65 Gew.-% bzw. mindestens 10 Gew.-% enthält.

22. Verfahren zur nicht therapeutischen kosmetischen Behandlung zum Schutz der Haut, der Kopfhaut, der Haare, der Wimpern, der Augenbrauen, der Nägel und der Schleimhäute, **dadurch gekennzeichnet, daß** auf diese in einer wirksamen Menge eine Zusammensetzung nach einem der Ansprüche 1 bis 21 aufgebracht wird.

## Claims

1. Cosmetic composition intended for the treatment of keratinous material, **characterized in that** it comprises, in a cosmetically acceptable aqueous support, at least one polysaccharide and an acrylic terpolymer comprising:
a) 20 to 70% by weight of a carboxylic acid containing α, β-monoethylenic unsaturation;
b) at least 20% by weight of a non-surfactant monomer containing monoethylenic unsaturation, which is different from a), and
c) 0.5 to 60% by weight of a nonionic urethane monomer which can be obtained by reacting a monohydric nonionic surfactant with a monoisocyanate containing monoethylenic unsaturation.

2. Composition according to Claim 1, **characterized in that** the carboxylic acid containing α,β-monoethylenic unsaturation a) is chosen from acrylic acid, methacrylic acid, itaconic acid and maleic acid.

3. Composition according to Claim 2, **characterized in that** the carboxylic acid containing α,β-monoethylenic unsaturation a) is methacrylic acid.

4. Composition according to any one of Claims 1 to 3, **characterized in that** the non-surfactant monomer containing monoethylenic unsaturation b) is chosen from C₁-C₄ alkyl acrylates and methacrylates, styrene, vinyltoluene, vinyl acetate, acrylonitrile and vinylidene chloride.

5. Composition according to Claim 4, **characterized in that** the non-surfactant monomer containing monoethylenic unsaturation is methyl or ethyl acrylate.

6. Composition according to any one of Claims 1 to 5, **characterized in that** the monohydric nonionic surfactant which can be used to obtain the nonionic urethane monomer c) has the formula: 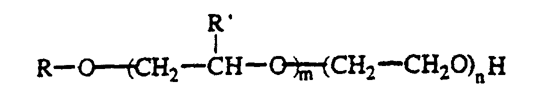 in which R is a C₆-C₃₀ alkyl or C₈-C₃₀ aralkyl group, R' is a C₁-C₄ alkyl group, n is an average number ranging from 5 to 150 and m is an average number ranging from 0 to 50, with the condition that n is at least as large as m and that n+m = 5-150.

7. Composition according to Claim 6, **characterized in that** R is chosen from dodecyl, C₁₈-C₂₆ alkyl and (C₈-C₁₃) alkylphenyl radicals and m = 0.

8. Composition according to any one of Claims 1 to 7, **characterized in that** the monoisocyanate containing monoethylenic unsaturation which can be used to form the nonionic urethane monomer c) is α,α-dimethyl-m-isopropenylbenzyl isocyanate.

9. Composition according to any one of Claims 1 to 8, **characterized in that** the acrylic terpolymer is obtained as an aqueous dispersion from methacrylic acid as component a), methyl acrylate as component b) and a nonionic urethane macromonomer of the following structure as component c): 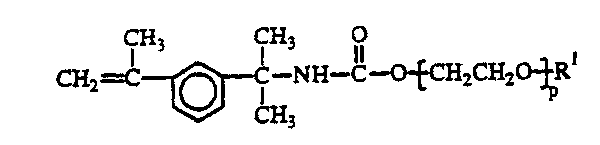 in which p ranges from 6 to 150 and R¹ is a C₁₈-C₂₆, preferably C₂₀-C₂₄, linear alkyl radical of plant origin, such as the docosyl radical.

10. Composition according to any one of Claims 1 to 9, **characterized in that** the acrylic terpolymer is present in concentrations ranging from 0.01 to 20% by weight, and preferably from 0.1 to 10% by weight, relative to the total weight of the composition.

11. Composition according to any one of Claims 1 to 10, **characterized in that** the polysaccharides are chosen from glucans, modified or non-modified starches (such as those extracted, for example, from cereals such as wheat, corn or rice, vegetables such as haricot beans, tubers such as potato or cassava), amylose, amylopectin, glycogen, dextrans, β-glucans, celluloses and their derivatives (methylcelluloses, hydroxyalkylcelluloses, ethylhydroxyethylcelluloses, carboxymethylcelluloses), fructosans, inulin, levane, mannans, xylans, arabans, galactans, galacturonans, chitin, glucuronoxylans, arabinoxylans, xyloglucans, galactomannans, glucomannans, pectic acids and pectins, alginic acid and alginates, arabinogalactans, carrageenans, agars, glycosaminoglucans, gum arabics, gum tragacanths, ghatti gums, karaya gums, carob gums, guar gums and xanthan gums.

12. Composition according to Claim 11,
**characterized in that** the polysaccharides are chemically modified or non-modified nonionic guar gums.

13. Composition according to Claim 12,
**characterized in that** the guar gums are modified with C₁-C₆ hydroxyalkyl groups.

14. Composition according to Claim 13,
**characterized in that** it contains a guar gum modified with a hydroxypropyl group.

15. Composition according to any one of Claims 1 to 14, **characterized in that** the polysaccharides is (are) present in concentrations ranging from 0.01 to 20% by weight, and preferably from 0.1 to 10% by weight, relative to the total weight of the composition.

16. Composition according to any one of Claims 1 to 15, **characterized in that** it has a pH ranging from 3.5 to 11, preferably from 5.5 to 11 and even more preferably from 5.5 to 8.5.

17. Composition according to any one of Claims 1 to 16, **characterized in that** the cosmetically acceptable aqueous medium consists of water or of water and at least one organic solvent chosen from the group consisting of hydrophilic, lipophilic and amphiphilic organic solvents or mixtures thereof.

18. Composition according to any one of Claims 1 to 17, **characterized in that** it also comprises at least one common cosmetic adjuvant chosen from fatty substances, standard gelling agents and/or thickeners, surfactants, moisturizers, emollients, sunscreens, hydrophilic or lipophilic active agents such as ceramides, anti-free-radical agents, sequestering agents, antioxidants, preserving agents, acidifying or basifying agents, fragrances, fillers, dyestuffs, silicones and reducing agents.

19. Composition according to any one of Claims 1 to 18, **characterized in that** it is in the form of an emulsion, a lotion, a gel, a vesicle dispersion, a paste or a solid stick or is packaged as an aerosol and is in the form of a mousse or a spray.

20. Composition according to any one of Claims 1 to 19, **characterized in that** it is used as a rinse-out or leave-in hair product to wash, dye, care for, condition or straighten the hair, to maintain the hairstyle or to permanently or temporarily reshape the hair, or as an antisun composition.

21. Composition according to any one of Claims 1 to 20, **characterized in that** the terpolymer comprises the monomers a), b) and c) in a proportion of 25% to 55%, 30% to 65% and at least 10% by weight, respectively.

22. Cosmetic, non-therapeutic treatment process for protecting the skin, the scalp, the hair, the eyelashes, the eyebrows, the nails and mucous membranes, **characterized in that** an effective amount of a composition as defined according to any one of Claims 1 to 21 is applied to these materials.
